# EUROPEAN PATENT APPLICATION

(11) **EP 4 306 130 A1**
(43) Date of publication of application: **17.01.2024**
(21) Application number: 22184128.1
(22) Date of filing: 11.07.2022
(51) Int. Cl.: A61K 47/69, A61K 9/00, A61P 31/06

(54) **INHALABLE FORMULATION FOR USE IN THE TREATMENT OF BACTERIAL LUNG INFECTIONS**

(71) Applicant: Solmic Biotech GmbH, 40225 Düsseldorf (DE)
(72) Inventor: The designation of the inventor has not yet been filed
(74) Representative: Schüssler, Andrea

(57) **Abstract**

The present invention concerns a nanocarrier formulation for use in the treatment of bacterial infections of the lower airways by inhalation therapy.

## Description

### 1. FIELD OF THE INVENTION

The present invention concerns a nanocarrier formulation for use in the treatment of bacterial infections of the lower airways by inhalation administration.

### 2. DISCUSSION OF THE RELATED ART

For treating bacterial lung infections, e.g. tuberculosis, a number of antibiotics have been developed. However, some of them show a side effect in the patient that is called "drug-induced phospholipidosis" (DIPL). Organs affected by phospholipidosis exhibit inflammatory reactions and histopathological changes. Phospholipidosis induced by drugs with a cationic amphiphilic structure is a generalized condition in humans and animals that is characterized by an intracellular accumulation of phospholipids and the concurrent development of concentric lamellar bodies. The primary mechanism responsible for the development of phospholipidosis is an inhibition of lysosomal phospholipase activity by the drugs. While the biochemical and ultrastructural features of the condition have been well characterized, much less effort has been directed towards understanding whether the condition has adverse effects on the organism. While this class of drugs should technically be referred to as cationic lipophilic, the term "cationic amphiphilic" is widely used and recognized in this field, and for this reason, the terminology cationic amphiphilic drugs (CADs) will be employed. In addition, a high percentage of CAD compounds also show inhibition of the potassium ion channel encoded by the human ether-à-go-go-related gene (hERG). Inhibition of hERG channels results in QT interval prolongation, which can result in life threatening ventricular tachyarrhythmia (Sun et al., Bioorg. Med. Chem. Lett. 2013: 23(16), pp. 4587-4590).

### 3. SUMMARY OF THE INVENTION

Thus, the object of the present invention is to provide a pharmaceutical formulation for treating bacterial lung diseases which reduces or even inhibits the induction of DIPL and/or inhibition of the potassium ion channel encoded by hERG.

This object is solved by the subject-matter according to claim 1. Preferred embodiments are the subject-matter of the dependent claims.

Inhalation, in particular pulmonary, therapy of anti-bacterial agents offers the potential to achieve higher local drug concentrations in the lower airways at the site of infection, while overall reducing systemic adverse effects. In the case of CADs, inhalation administration reduces systemic drug concentrations thereby achieving both a reduction in CYP3A4 metabolism and a reduced QT prolongation. As a consequence, the scope for co-administration of CADs with other therapeutic agents might be significantly broadened.

Due to the poor aqueous solubility of many anti-bacterial agents, in particular bedaquiline, a conventional dry powder formulation for inhalation, specifically in the form of an interactive powder mixture, i.e. a micronized drug dispersed on the surface of a lactose carrier particle, might not be appropriate, as there is increasing evidence that inhaled dry powders of poorly soluble compounds are associated with adverse effects in the lower airways, in particular in the lung, including particulate accumulation in the lower airways (e.g. lung), increased macrophage numbers, increased prevalence of foamy macrophages and particle-induced inflammation (Forbes et al., Adv. Drug Deliv. Rev. 2014; 71, pp.15-33). To circumvent this issue, the delivery via cell-targeted nanocarriers has been implemented to encapsulate therapeutically relevant concentrations of the anti-bacterial agent for administration as either a nebulized liquid or a spray-dried dry powder formulation. However, one significant clinical disadvantage of spray-dried powder formulations (as described by e.g. Huck, et al., Adv Healthcare Mater, 2022, 11(11), 2102117) is the amount of additional excipient required to create a respirable dry powder, which ultimately limits the amount of antibiotic able to be inhaled into the lung. Nebulized nanocarrier formulations, as described here, are not limited in this regard and can be dosed at significantly higher concentrations, which is necessary for effective bacterial eradication and prevention of resistance development.

In general, cell-targeted nanocarriers are a suitable vehicle for specifically delivering encapsulated compounds to (a) given cell type(s) expressing the respective targeting structure. When employed therapeutically, such technology highly focuses in delivering compound to the cell type(s) of choice. As an example, it has been shown earlier that the compounds needed to be delivered by targeted liposomes selectively reached the cell type of choice, such as myeloid dendritic cells (mDCs), and delivered their contents into such cells (Gieseler RK et al., Scand J Immunol. 2004; 59:415-24; WO 2005/092288 A1).

Thus, the present invention concerns an inhalable formulation comprising an active agent complex comprising one or more anti-bacterial agents encapsulated in a DMPG-based or DMPC-based nanocarrier and at least one cell-specific targeting ligand on the surface of the nanocarrier, wherein the targeting ligand specifically binds to a receptor on the surface of a carbohydrate recognition domain (CRD)-positive cell of the lower airways for use in a method of treating a bacterial infection of the lower airways.

### 4. BRIEF DESCRIPTION OF THE FIGURES

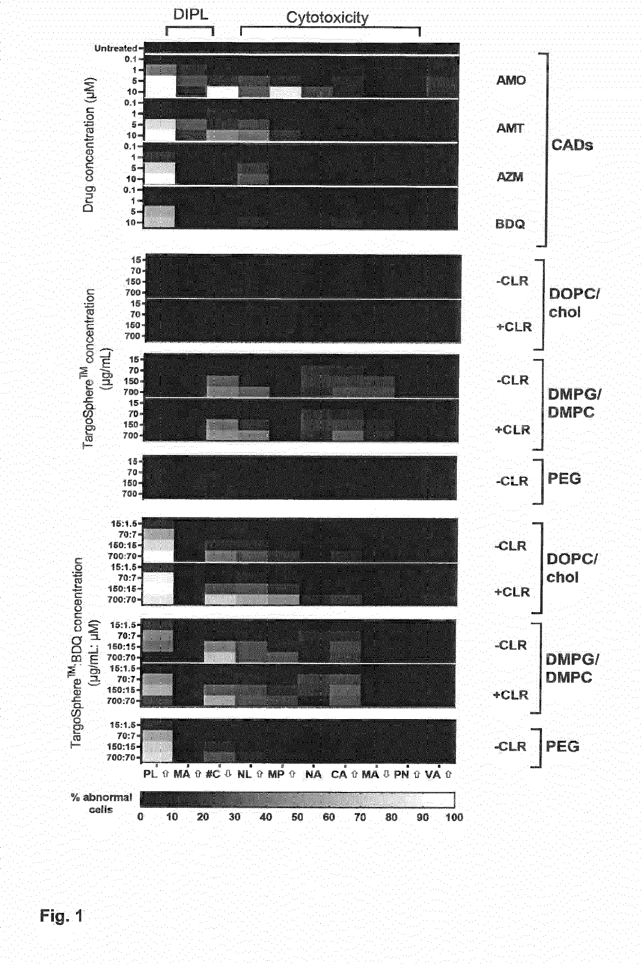

- Fig. 1:: A macrophage-like cell line (J774 cells) were used to screen whether three different nanocarrier formulations differing in their lipid compositions (1: DOPC/Chol, 2: DMPC/DMPG or 3: pegylated DOPC/Chol) i) are cytotoxic to this cell type and protect the cells from BDQ-induced DIPL. A heat map was used to depict the number of cells with DIPL-induced abnormalities (first two left-hand columns) and cytotoxicity markers (columns on the right). Black fields indicate that < 10% of the cells in the treatment groups were abnormal, whereas grey-to-white colored fields denote an increasing proportion of cells with abnormalities. The heatmap at the top depicts the positive control group for DIPL, which consisted of four known CADs (amiodarone, amytryptiline, azithromycin, and BDQ) administered to the cells in solution (no formulation). The white fields in the left column show that nearly 100% cells exhibit an abnormal phospholipid accumulation after treatment with CADs, which is expected. The heatmap in the middle shows the negative control group consisting of the three different lipid nanocarrier systems (without drug) both with and without fucose targeting moieties on the surface (+CLR or -CLR, respectively). The predominantly black fields denote that the empty nanocarriers do not induce DIPL and have low cytotoxicity. The heatmap at the bottom shows the same nanocarriers loaded with BDQ. The nanocarriers do not fully inhibit DIPL occurrence, but in the case of the DMPG/DMPC formulation, the number of cells with DIPL is reduced by the formulation. The numbers on the left-hand side of each heatmap denote the CAD dose (µM) and the total lipid concentration (µg/mL) added to each well. The values depicted the mean % abnormal cells from six replicate formulations tested in four different cell passage numbers.

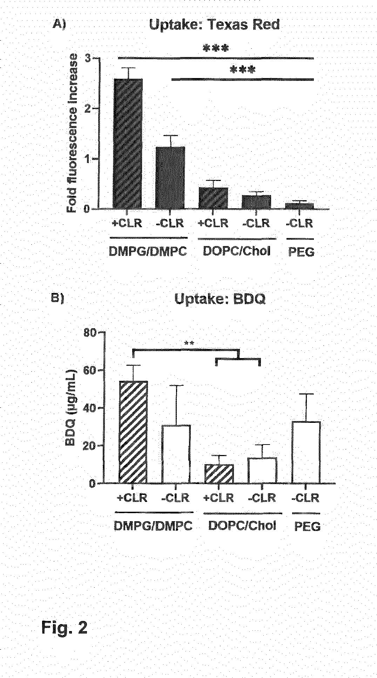

- Fig. 2:: Uptake of the fluorescent marker, Texas Red, and BDQ in J774A.1 cells following 48 h incubation with anionic, neutral or pegylated nanocarrier (TargoSpheres^{™}), both with and without targeting ligand (TLR). A single TargoSpheres^{™} batch was assessed in four replicate cell passage numbers. Values represent the mean ± standard deviation (n=4). **p<0.01; ***p<0.001.

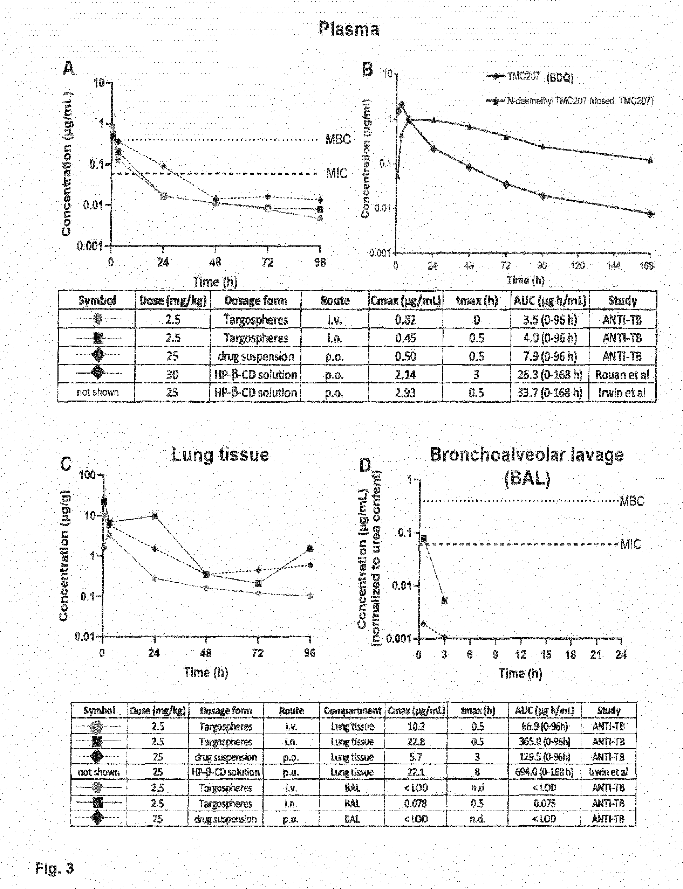

- Fig. 3:: Pharmacokinetic profiles of BDQ-loaded DMPG/DPMC nanocarriers (+CLR; labelled as TargoSpheres^{™}) in healthy mice (Balb-c, n=6 per group per time point). A) The plasma concentration was measured following a single intravenous (i.v., circles) or intranasal (i.n., a form of intrapulmonary administration, squares) administration of the nanocarriers. As a reference, non-formulated BDQ powders were administered via per oral gavage (diamonds, dashed line) at a dose comparable to a study by Rouan et al. (Antimicrob Agents Chemother, 2012, 1444-51; diamonds + solid line). B) The plasma concentration of per orally dosed BDQ (also known as TMC207) and its main metabolite are taken from Rouan et al. (2012) and depicted for comparison to the non-formulated control BDQ results of the current study. The table reports the primary pharmacokinetic parameters from the current study and the literature. The BDQ concentration in lung tissue (C) and bronchial alveolar lavage fluid (D) following i.v. and in. administration of BDQ-loaded nanocarriers versus p.o. administration of the non-formulated BDQ. Taken in total, the pharmacokinetic data show that intrapulmonary administration of the nanocarrier formulation leads to lower systemic exposure compared to per oral administration and higher lung BDQ concentrations.

### 5. DETAILED DESCRIPTION OF THE PREFERRED EMBODIMENTS

Thus, the present invention concerns an inhalable formulation comprising an active agent complex comprising at least one anti-bacterial agent encapsulated in a DMPG- or DMPC-based nanocarrier having at least one cell-specific targeting ligand on its surface, wherein the targeting ligand specifically binds to a receptor on the surface of a CRD-positive cell or CLR-positive cell of the lower airways for use in a method of treatment of a bacterial infection of the lower airways.

In a preferred embodiment the present invention concerns a lipid-drug complex comprising an anti-bacterial agent encapsulated in a DMPG- or DMPC-based liposome and at least one carbohydrate or derivative thereof as a targeting ligand on the outer surface of the liposome, wherein the targeting ligand specifically binds to a receptor expressing C-type lectin-like carbohydrate recognition domains (CRD or CLR) or other receptors with CRD or CLR on the surface of a cell of the lower airways.

In a preferred embodiment the active agent complex is part of a pharmaceutical composition or combination combined with a pharmaceutically acceptable carrier.

The present invention enables the preferentially delivery of the anti-bacterial agent as an active agent to a CLR- positive or CRD-positive cell of a mammalian subject, including a human.

In the present application the terms "nanocarrier", "active agent complex", "active agent formulation" and "active agent system" are used interchangeably and are used synonymously to each other.

A "complex" or "formulation" is a mixture or adduct resulting from chemical binding or bonding between and/or among its constituents or components, including the lipid, active agent, and other optional components. Chemical binding or bonding can have the nature of a covalent, ionic, hydrogen, van der Waal's, or hydrophobic bond, or any combination of these bonding types linking the constituents of the complex at any of their parts or moieties, of which a constituent can have one or a multiplicity of moieties of various sorts. Not every constituent of a complex or formulation needs to be bound to every other constituent, but each constituent has at least one chemical binding or bonding with at least one other constituent of the complex. In accordance with the present invention, examples of lipid-active agent complexes include liposomes (lipid vesicles), or lipid-active agent sheet-disk complexes. Also, lipid-conjugated active agents can be a part of the lipid-active agent complex.

Useful techniques for making lipid-active agent formulations, such as liposomes, are known in the art (e.g., Sullivan SM et al., Antisense Res Dev 1992;2:187-97; Laverman P et al., Crit Rev Ther Drug Carrier Syst 2001;18:551-66; Oussoren C, Storm G, Adv Drug Deliv Rev 2001;50:143-56; U.S. Patent No. 5,773,027; U.S. Patent No. 5,223,263; WO 96/10399 A1; Bailey-Hytholt, C. M., et al. Formulating and Characterizing Lipid Nanoparticles using a Microfluidic Mixing Platform., J. Vis. Exp. (168), e62226, doi:10.3791/62226 (2021).).

For cellular targeting, in accordance with the present invention, liposomes or lipid-based nanocarriers can be prepared by any of the methods described above. In accordance with the present invention, the lipids are DMPG [1,2-Dimyristoyl-sn-glycero-3-phosphoglycerol (preferably sodium salt)] and/or DMPC [1,2-Dimyristoyl-sn-glycero-3-phosphocholine]. In a particular preferred embodiment a combination of both is used, e.g. in a 50:50 ratio or with a vast majority of DPMG (e.g. 75%-80% DPMG: 20%-25% DPMC). Optionally, other lipids such as steroids, cholesterol, aliphatic amines (e.g. long-chained aliphatic amines or carboxylic acids), long-chained sulfates and phosphates, diacetyl phosphate, butylated hydroxytoluene, tocopherols, retinols, and isoprenoid compounds can be intermixed with the phospholipid components to confer certain desired and known properties on the formed vesicles. In addition, synthetic phospholipids containing either altered aliphatic portions such as hydroxyl groups, branched carbon chains, cyclo-derivatives, aromatic derivatives, ethers, amides, polyunsaturated derivatives, halogenated derivatives, or altered hydrophilic portions containing carbohydrate, glycol, phosphate, phosphonate, quaternary amine, sulfate, sulfonate, carboxy, amine, sulfhydryl, or imidazole groups and combinations of such groups can be intermixed with the above-mentioned phospholipids and used in accordance with the invention.

The use of an organic solvent can facilitate the production of the lipid-active agent formulation. After mixing the active agent and lipids, the organic solvent is removed by, e.g., evaporating by vacuum, application of heat, or hot ethanol injection (e.g. United States Patent No. 4,515,736), as long as the lipid and active agent are stable at the temperature used. Dialysis and/or chromatography (e.g. affinity chromatography) can also be employed to remove the organic solvent. Hydrating the active agent is performed with water or any biocompatible aqueous buffer (e.g., phosphate-buffered saline, HEPES, or TRIS) that maintains physiological osmolarity. Rehydration of liposomes can be accomplished simultaneous to remove the organic solvent, or alternatively, can be delayed until a more convenient time for using the liposomes (e.g. US Patent No. 4,235,871). The shelf-life of hydratable ("dry") liposomes - typically 8-12 months - can be increased by lyophilization.

In one embodiment of the present invention, the lipid-active agent complex is a unilamellar liposome. Such liposomes accommodate higher levels of active agent which may interact with inner and outer surfaces of the liposomes. However, in accordance with the present invention multilamellar liposomes and/or PEGylated liposomes can also be used.

The lipid-active agent complex is preferably, but not necessarily,∼30-200 nm in diameter.

In accordance with the present invention, lipid-active agent complexes can be preserved by any known method, such as lyophilization (e.g., Crowe et al., United States Patent No. 4,857,319). Typically, lyophilization or other useful cryopreservation techniques involve the inclusion of a cryopreservant, such as a disaccharide.

The targeting ligand can be a small chemical compound, an antibody, a sugar, a peptide or an aptamer. As used herein, the term "antibody" (Ab) is meant to include intact immunoglobulin molecules as well as antibody fragments (such as, for example, Fab and F(ab')2 fragments) which are capable of specifically binding to a surface receptor. Fab and F(ab')2 fragments lack the Fc fragment of intact antibody. Moreover, antibodies useful for the purposes of the present invention include chimeric, single chain, multifunctional (e.g. bispecific) and humanized antibodies or human antibodies.

The present invention uses nanocarriers, particularly liposomes, having targeting ligands on their inner and/or outer surface. These targeting ligands provide a surface labeling of the nanocarrier. For purposes of the present invention the targeting ligand is attached to the lipid component (e.g. a liposome) by techniques known in the art (WO 05/092288 A1), preferably through covalent binding. Preferred examples of targeting ligands are those specifically binding to a receptor expressing C-type lectin-like carbohydrate recognition domains (CRD or CLR). Examples are monofucose, polyfucose or derivatives thereof. One particular preferred targeting ligand is cholesten-5-yloxy-N-(4-((1-imino-2-D-thiofucosylethyl)amino)alkyl)formamide (Fuc-C4-chol), cholesteryl-(4-(3-((2R,3S,4R,5S,6S)-3,4,5-trihydroxy-6-(hydroxymethyl)tetrahydro-2H-pyran-2-yl)thioureido)butyl)carbamate (Thiourea-Galac-C4-chol). In a preferred embodiment, fucosylated (Fuc) liposomes are prepared by means of a linkage of fucosyl targeting ligand, may be to a spacer that is attached to a suitable lipophilic membrane anchor on the liposomes or may be to a distal end of the anchor on the liposomes or directly to the lipid component of the liposomes. Suitable compounds are polyethylene glycol (PEG) or hydroxyethyl starch (HES).

CLRs function as 'pathogen entry ports' into a cell expressing such receptors. As to their biological function, the endocytic members of APC-expressed CLRs serve for the uptake of pathogens and their intracellular break-down, subsequent controlled processing of pathogen-derived protein antigens to 10-mer or 12-mer peptides and the association of such antigens with class I or class II proteins of the APC's major histocompatibility complex (MHC). These combinatorial molecules allow for MHC-restricted - i.e., strongly controlled - presentation of the antigens on the APC surface where instructing, in a delicately balanced concert with other surface molecules and soluble signals, other immune cells to elicit a protective cellular and/or humoral immune response against the pathogen initially internalized. Importantly, various pathogens have evolved to resist intracellular degradation by APCs.

As the nanocarriers of the present invention can be scaled at diameters of between approx. 50-200 nm - hence falling in the nanocarrier category - they are sized in the range of most bacteria and viruses. Like pathogens, the nanocarriers of the present invention are internalized in clathrin-dependent manner whereupon both carrier and payload (= anti-bacterial agent; e.g. bedaquiline) are delivered into endosomes. Furthermore, and again like reservoir-forming pathogens, a given therapeutic payload can escape the endo(lyso)somal compartment to access the cytoplasm.

Targeted CRD-positive cells or CLR-positive cells (both terms are used interchangeably) include, but are not limited to, airway epithelial cells (e.g. lung vascular cells), and immune cells like macrophages (e.g. alveolar macrophages).

The present invention is directed to target CRD-positive cells or CLR-positive cells of the lower airways which are lined with respiratory mucosa or respiratory epithelium (also called airway epithelial cells). The lower airways (also called "lower respiratory tract") includes the portion of the larynx below the vocal folds, trachea, bronchi and bronchioles. The lungs are also part of the lower respiratory tract and include the respiratory bronchioles, alveolar ducts, alveolar sacs, and alveoli.

The terms "compound(s)", "pharmaceutical", "agent" or "drug" according to the present disclosure include their tautomers, stereoisomers and mixtures thereof and the salts thereof, in particular the pharmaceutically acceptable salts thereof, and the solvates and hydrates of such compounds, including the solvates and hydrates of such tautomers, stereoisomers and salts thereof.

The terms "treat," "treatment," and "treating" embraces therapeutic, i.e. curative and/or palliative, treatment. Thus, the terms "treatment" and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

When this disclosure refers to patients requiring treatment, it relates primarily to treatment in mammals, in particular humans.

By "therapeutically effective amount" or "pharmaceutically effective amount" is meant a compound or compounds, as disclosed for this invention, which has a therapeutic effect. The doses of compounds of the present disclosure which are useful in treatment are therapeutically effective amounts. Thus, as used herein, a therapeutically effective amount means those amounts of compounds which produce the desired therapeutic effect as judged by clinical trial results and/or model animal infection studies. In particular embodiments, the compounds are administered in a pre-determined dose, and thus a therapeutically effective amount would be an amount of the dose administered. This amount and the amount of the compound can be routinely determined by one of skill in the art, and will vary, depending on several factors, such as the particular microbial strain involved. This amount can further depend upon the patient's height, weight, sex, age and medical history.

A "therapeutic effect" relieves, to some extent, one or more of the symptoms of the infection, and includes, to some extent, curing an infection. "Curing" means that the symptoms of active infection are eliminated, including the total or substantial elimination of excessive members of viable microbes of those involved in the infection to a point at or below the threshold of detection by traditional measurements. However, certain long-term or permanent effects of the acute or chronic infection may exist even after a cure is obtained (such as extensive tissue damage). As used herein, a "therapeutic effect" is defined as a statistically significant reduction in bacterial load in a host, Improvement of pulmonary function, or improvement in infection symptoms or functional status.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

The term "pharmaceutically acceptable salt" refers to salts that retain the biological effectiveness and properties of the compounds and, which are not biologically or otherwise undesirable. In many cases, the compounds are capable of forming acid and/or base salts by virtue of the presence of amino and/or carboxyl groups or groups similar thereto.

The term "administration" or "administering" refers to a method of giving a dosage of a pharmaceutical composition to a mammal, for example, by inhalation. The method of administration can vary depending on various factors, e.g., the components of the pharmaceutical composition, the site of the potential or actual bacterial infection, e.g. the lungs, the microbe involved, and the severity of an actual microbial infection.

The term "microbial infection" or "bacterial infection" refers to the undesired proliferation or presence of invasion of pathogenic microbes in a host organism. This includes the excessive growth of microbes that are normally present in or on the body of a mammal or other organism, e.g. in the lungs. More generally, a microbial infection can be any situation in which the presence of a microbial population(s) is damaging to a host mammal. Thus, a microbial infection exists when excessive numbers of a microbial population are present in or on a mammal's body, or when the effects of the presence of a microbial population(s) is damaging the cells or other tissue of a mammal.

The term "sequentially" refers to the administration of more than one therapeutic agent at separate times. The therapeutic agents can be administered in any order. Unless the drugs are formulated together, they are considered to be administered sequentially. In one embodiment, two or more therapeutic agents are considered to be administered sequentially if they are administered within 24 hours of each other. In another embodiment, two or more therapeutic agents can be administered in less than a 24 hour period. In another embodiment, two or more therapeutic agents can be administered in less than a 12 hour period. In another embodiment, two or more therapeutic agents can be administered in less than a 6 hour period. In another embodiment, two or more therapeutic agents can be administered in less than a 3 hour period. In one embodiment, the therapeutic agents are administered immediately, one right after another or not more than 60 minutes apart from each other.

"One or more" and "at least one" encompasses one to ten (explicitly one, two, three, four, five, six, seven, eight, nine or ten) specimen.

Non-limiting examples of anti-bacterial agents that are active against bacterial infections of the lower airways, in particular to treat tuberculosis (TB), include bedaquiline (BDQ); aminoglycoside antibiotics (such as kanamycin A, amikacin, tobramycin, dibekacin, gentamicin, sisomicin, netilmicin, neomycin B, neomycin C, paromomycin and streptomycin); fluroquinolones (such as moxifloxacin, levofloxacin, sparfloxacin, nalidixic acid, ciprofloxacin, cinoxacin, oxolinic acid, piromidic acid, pipemidic acid, rosoxacin, enoxacin, fleroxacin, lomefloxacin, nadifloxacin, norfloxacin, ofloxacin, perfloxacin, rufloxacin, balofloxacin, grepafloxacin, pazufloxacin, temafloxacin, tosufloxacin, clinafloxacin, gatlifloxacin, sitafloxacin, prulifloxacin, delafloxacin, nemofloxacin, danofloxacin, difloxacin, enrofloxacin, ibafloxacin, marbofloxacin, orbifloxacin, sarafloxacin and trovafloxacin); oxazolidinone antibiotics (such as linezolid); and nitroimidazole antibiotics (such as metronidazole, tinidazole and nimorazole). In a particular preferred embodiment the anti-bacterial agent is bedaquiline. In one embodiment, the aminoglycoside antibiotic is amikacin. In one embodiment, the fluoroquinolone is selected from levofloxacin and moxifloxacin. In one embodiment, the nitroimidazole antibiotic is metronidazole.

In one embodiment, the pharmaceutical treatment includes administering an active agent complex comprising a lipid-drug complex comprising bedaquiline encapsulated in a DMPG- and/or DMPC-based nanocarrier alone or either sequentially or in combination with one or more encapsulated or non-encapsulated anti-bacterial agents like amikacin or linozolid.

It is contemplated by the present invention that the pharmaceutical treatment can be administered through inhalation.

For pulmonary administration, the upper airways are avoided in favour of the middle and lower airways. Pulmonary drug delivery can be accomplished by inhalation of a nebulized suspension of the active agent complex through the mouth and throat. Particles having a mass median aerodynamic diameter (MMAD) of greater than about 5 microns generally do not reach the lung; instead, they tend to impact the back of the throat and are swallowed and possibly orally absorbed. Particles having diameters of about 2 to about 5 microns are small enough to reach the upper- to mid- pulmonary region (conducting airways), but are too large to reach the alveoli. Smaller particles, i.e., about 0.5 to about 2 microns, are capable of reaching the alveolar region. Particles having diameters smaller than about 0.5 microns can also be deposited in the alveolar region by sedimentation, although very small particles may be exhaled.

In one embodiment, a nebulizer is selected on the basis of allowing the formation of an aerosol of the active agent complex disclosed herein having an MMAD predominantly between about 0.5 to about 5 microns. In one embodiment, the delivered amount of the active agent complex provides a therapeutic effect for respiratory infections, in particular tuberculosis, most preferred multi-resistant tuberculosis. The nebulizer can deliver an aerosol comprising a mass median aerodynamic diameter from about 0.5 microns to about 5 microns, a mass median aerodynamic diameter from about 1.0 microns to about 3.0 microns, or a mass median aerodynamic diameter from about 1.5 microns to about 2.5 microns. In some embodiments, the MMAD can be about 0.5 microns, about 1.0 microns, about 1.5 microns, about 2.0 microns, about 2.5 microns, about 3.0 microns, about 3.5 microns, about 4.0 microns, about 4.5 microns or about 5.0 microns. In one embodiment, the MMAD ranges from about 2.5 to about 5.0 microns. In another embodiment, the MMAD ranges from about 3.0 to about 4.5 microns. In some embodiments, the nebulizer can be a breath actuated nebulizer (BAN). In some embodiments, the aerosol can be produced using a vibrating mesh nebulizer. An example of a vibrating mesh nebulizer includes the PARI E-FLOW^{®} nebulizer or a nebulizer using PARI eFlow technology. More commercial examples of nebulizers that can be used with the formulations described herein include Respirgard II^{®}, Aeroneb^{®}, Aeroneb Pro^{®}, Aeroneb Go^{®}, AERx^{®}, AERx Essence^{®}, Porta-Neb^{®}, Freeway Freedom^{®}, Sidestream^{®}, Ventstream^{®}, I-neb^{®}, PARI LC- Plus^{®}, and PARI LC-Start^{®}. In one embodiment, the nebulizer is a breath actuated nebulizer.

The formulation can comprise a conventional pharmaceutical carrier, diluent, excipient, buffers, surfactants or the like that are approved for inclusion in inhaled products per the US National Formulary and database of approved excipients maintained by USFDA and other regulatory agencies. Non-limiting examples of carriers and excipients include, e.g., water, ethanol, glycerin, propylene glycol, PEG 1000, sorbitan trioleate, soya lecithin, lecithin, oleic acid, magnesium stearate, sodium lauryl sulfate, lactose, mannitol, dextrose, methylparaben, propylparaben, chlorobutanol, benzalkonium chloride, cetylpyridinium chloride, thymol, ascorbic acid, sodium bisulfate, sodium metabisulfite, sodium bisulfate, EDTA, NaOH, tromethamine, ammonia, HCl, H₂S0₄ , HN0₂, citric acid, CaCl₂ and CaCO₃ .

Liquid pharmaceutically administrable compositions can, for example, be prepared by dissolving, dispersing, etc. of the active agent complex as defined above and optional pharmaceutical adjuvants in a carrier (e.g., water, saline, aqueous dextrose, glycerol, glycols, ethanol or the like) to form a solution or suspension. Solutions to be aerosolized can be prepared in conventional forms, either as liquid solutions or suspensions, as emulsions, or in solid forms suitable for dissolution or suspension in liquid prior to aerosol production and inhalation. The percentage of active compound contained in such aerosol compositions is highly dependent on the specific nature thereof, as well as the activity of the compound and the needs of the subject. However, percentages of active ingredient(s) of about 0.01% to about 90% in solution are employable.

The formulation may be administered at a therapeutically effective dosage, e.g., a dosage sufficient to provide treatment for the disease states previously described. The amount of active compound administered will, of course, be dependent on the subject and disease state being treated, the severity of the affliction, the manner and schedule of administration and the judgment of the prescribing physician.

In one embodiment, the formulations, with one or more therapeutic agents, can be administered to the lungs in less than about 60 minutes, about 55 minutes, about 50 minutes, about 45 minutes, about 40 minutes, about 35 minutes, about 30 minutes, about 25 minutes, about 20 minutes, about 15 minutes, about 10 minutes, about 5 minutes, about 4 minutes, about 3 minutes, about 2 minutes, and about 1 minute.

Methods and compositions described herein can be used to treat pulmonary infections and disorders of the lower airways. Examples of other such disorders can include tuberculosis, cystic fibrosis, pneumonia, and chronic obstructive pulmonary disease, including chronic bronchitis, and some asthmas, wherein tuberculosis, in particular multi-drug resistant tuberculosis, is preferred. Some embodiments include treating an infection comprising one or more bacteria selected from the group consisting of *Pseudomonas aeruginosa, Pseudomonas fluorescens, Pseudomonas acidovorans, Pseudomonas alcaligenes, Pseudomonas putida, Bordetella pertussis, Bordetella parapertussis, Bordetella bronchiseptica, Haemophilus influenzae, Haemophilus parainfluenzae, Legionella pneumophila, Burkholderia cepacia, Streptococcus pneumoniae* and *Mycobacterium tuberculosis.*

It could be shown in the present invention that DMPC-or DMPC-based nanocarrier formulations, in particular when having Fuc-C4-Chol as a CLR targeting ligand on its surface, were superior to neutral and pegylated formulations for delivering antibacterial agents, particularly BDQ, to the intracellular environment of macrophage-like cells and, despite a higher level of cell stress markers, which might be explained by more intensive interactions of the anionic DMPC-based and/or DMPC-based nanocarrier with the cells, they also appeared to reduce the number of cells with pronounced DIPL in the cell population, as compared to neutral and pegylated nanocarriers. Based on the results of the examples it is justified to postulate that higher anti-bacterial agent, e.g. BDQ, concentrations in the lungs lead locally to a steeper concentration gradient. This gradient drives drug diffusion through the lungs and is believed to increase levels of active drug in hard-to-reach compartments such as the fibrotic granuloma capsule. This should lead to an increase in therapy efficiency with a reduction in the development of resistance.

One further advantage of inhaling nebulized BDQ-nanocarriers would be bypassing the first passage through the liver (the so-called "first-pass effect") and thus delayed metabolization of the BDQ to N-desmethyl-BDQ. The encapsulation of the BDQ could further prevent or delay distribution processes such as protein and albumin binding as well as diffusion processes in other tissues, which could lead to altered biodistribution in the body and pharmacokinetics. The BDQ metabolite N-desmethyl-BDQ is primarily formed in the liver by cytochrome P450 34A biotransformation and has a 3- to 5-fold attenuated activity against *Mycobacterium tuberculosis* strains compared to BDQ. The metabolite is also responsible for a major proportion of systemic side effects - such as phospholipidosis and "QT prolongation".

The following examples are to be understood as exemplary and not as limiting the scope of the invention. The use of the anti-bacterial agent bedaquiline (BDQ) in the examples is to be understood as an exemplary compound for a number of suitable antibacterial compounds as mentioned above.

### 6. EXAMPLES

### Example 1: Preparation of nanocarriers encapsulating bedaquiline (BDQ)

BDQ liposomal TargoSphere^{®} formulations were prepared via the thin-film hydration method followed by extrusion. First, lipids, BDQ and targeting ligand (Fuc-C4-Chol) were dissolved in chloroform, ethanol or a mixture of water and ethanol, respectively. Then, stock solutions were combined in a round-bottom flask according to mole fractions shown in Table 1 and the solvents were slowly removed using a rotary evaporator to obtain a homogenous film. To get rid of any residual solvent the films were dried in vacuum for two days. For lipid-based nanocarrier production the dry films were hydrated with PBS to a film concentration of 40 mg/mL. The crude sample was then extruded through a polycarbonate membrane (Whatman^{®} Nuclepore^{™} Track-Etched Membrane) with a pore size of 200 nm followed by an extrusion through 50 nm.

**Table 1: Composition of different TargoSphere^{®} liposomal formulations.**

| Composition abbreviation | Component | Mole fraction |
|---|---|---|
| DMPC/DMPG | DMPG | 74.66 % |
| | DMPC | 8.16 % |
| | Fuc-C4-Chol | 8.00 % |
| | BDQ | 9.18% |
| DOPC/Chol | DOPC | 67.81 % |
| | Chol | 21.32% |
| | BDQ | 10.87 % |
| PEG-DOPC/Chol | DOPC | 66.51 % |
| | Chol | 18.49 % |
| | m-PEG-2000-DSPE | 5.00 % |
| | BDQ | 10.00 % |

DMPG [1,2-Dimyristoyl-sn-glycero-3-phosphoglycerol (preferably sodium salt)], DMPC [1,2-Dimyristoyl-sn-glycero-3-phosphocholine], DOPC [1,2-dioleoyl-sn-glycero-3-phosphocholine], Chol [Cholesterol], m-PEG-2000-DSPE [N-(carbonyl-methoxypolyethylene glycol-2000)-1,2-distearoyl-sn-glycero-3-phosphoethanolamine, sodium salt]

### Example 2: In-vitro assessment of respirable bedaquiline-loaded nanocarriers designed for inhalation therapy of multidrug-resistant tuberculosis

High content analysis was initially used as a rapid screening tool to evaluate interactions between lipid-based nanocarrier (TargoSphere^{®}) formulations applied as liquid dispersions and macrophage cells. Cells were treated with five different formulations (n=6 batches) containing BDQ encapsulated within liposomal formulations of different lipid compositions (DOPC/Chol, PEG-DOPC/Chol, and DMPG/DMPC) as described in Example 1. Two of the formulations (DOPC/Chol and DMPG/DMPC) contained Fuc-C4-Chol as a C-type lectin receptor targeting ligand (+CLR). Four concentrations ranging from 15-700 µg/mL (total solid content) were tested. After an incubation time of 48 h three assays with different fluorescence markers were performed. For the assessment of cell health and morphology, cells were stained with 100 µl of a dye cocktail containing Hoechst 33342 (10 mg/ml, Thermo Fischer, Dreieich, Germany), MitoTracker^{®} (300 nM, Thermo Fischer, Dreieich, Germany) and Image-IT^{™} DEAD Green^{™} (35 nM, Thermo Fischer, Dreieich, Germany) for 30 min at 37°C. After a washing step with 100 µl PBS cells were fixed with 100 µl 4 % paraformaldehyde for 15 min at room temperature in the dark. Fixed cells were stained overnight with HSC CellMask^{™} Deep Red (10 mg/ml, Thermo Fischer, Dreieich, Germany) after a second washing step. For the determination of the content of phospholipids, cells were incubated with HCS LipidTOX^{™} Red (Thermo Fischer, Dreieich, Germany) diluted 1:1000 (according to the manufacturer's protocol) alongside the treatment of the cells for 48 h. After a washing step with 100 µl PBS cells were fixed with a dye cocktail containing Hoechst 33342 (10 mg/ml, Thermo Fischer, Dreieich, Germany) and 4% paraformaldehyde for 30 min at room temperature in the dark. Cells were washed again and incubated with HCS LipidTOX^{™} Green (Thermo Fischer, Dreieich, Germany) diluted 1:1000 (according to the manufacturer's protocol) for additional 30 min at room temperature in the dark for detection of neutral lipids. Before imaging, cells were washed once with PBS. Both assays were stored at 4°C.

For high content analysis, images were documented using the Cytation^{™} 5 Cell Imaging Multi-Mode Reader (BioTek, Bad Friedrichshall, Germany) with a 40x objective in standard 2D imaging mode. For each well 6 pictures were analysed using the Gen5 software (BioTek, Bad Friedrichshall, Germany). To identify nucleated cells for cell health and morphology analysis Hoechst 33342 cell nuclear staining was used. MitoTracker^{®} accumulates in active mitochondria through the detection of changes in the mitochondrial membrane potential. Image-IT^{™} DEAD Green^{™} as an important impermeant dye to healthy cells that becomes permanent when the plasma membrane of cells is compromised. For both dyes the fluorescence intensity values were measured. HSC CellMask^{™} Deep Red dye was used to highlight the cytoplasmic regions within the cells. Area and size of the vacuoles were identified and measured based on negative staining. For the lipid assay, Hoechst 33342 was again used to identify the nucleated cells. HCS LipidTOX^{™} Green was used to detect the neutral lipids in the cell area, because of his high affinity. To detect and quantify the intercellular accumulation of phospholipids, also called phospholipidosis, HCS LipidTOX^{™} Red was used. Nine basic parameters were captured and characterised: Cell health (increased or reduced mitochondrial activity, membrane permeability), cell morphology (cell area, nuclear area, polynucleated cells) and vacuole properties (vacuole area, phospholipidosis, neutral lipid content). Each experiment was repeated with different passage numbers at least 6 times. For any plate six wells with untreated cells, three wells with amiodarone (phospholipidosis control) and three wells for FCCP (mitochondrial control) were carried along. If the cell number per well was lower compared to the untreated cells, the well was excluded. Criteria for the inclusion for each parameter of the cells is shown in Table 2. Limits were set with the double of the standard deviation of the mean compared to the untreated cells.

**Table 2: Basic parameters and inclusion criteria for high content analysis.**

| **#** | **Parameters** | **Inclusion criteria** |
|---|---|---|
| 1 | Elevated mitochondrial activity | Cells [%] > mean mitochondrial activity + 2^{nd} SD of the untreated population |
| 2 | Reduced mitochondrial activity | Cells [%] > mean mitochondrial activity + 2^{nd} SD of the untreated population |
| 3 | Elevated membrane permeability | Cells [%] > mean membrane permeability + 2^{nd} SD of the untreated population |
| 4 | Abnormal nuclear area | Cells [%] < mean - 2^{nd} SD nuclear area or > mean + 2^{nd} SD nuclear area of untreated cell population |
| 5 | Elevated cellular area | Cells [%] > mean cell area + 2^{nd} SD of the untreated population |
| 6 | Polynucleated cells | Cells [%] > nucleus number per cell |
| 7 | Elevated vacoule area | Cells [%] > mean vacoule area + 2^{nd} SD of the untreated population |
| 8 | Elevated neutral lipids | Cells [%] > mean neutral lipid content per cell + 2^{nd} SD of the untreated population |
| 9 | Elevated phospholipids | Cells [%] > mean phospholipid content per cell + 2^{nd} SD of the untreated population |

When cells were exposed to bedaquiline (BDQ)-loaded formulations, anionic DMPG/DMPC- nanocarrier formulations reduced somewhat the number of cells in the population with pronounced DIPL in comparison to neutral and pegylated formulations. This is an interesting observation considering that anionic nanocarrier-formulations showed a higher intracellular uptake of BDQ (and Texas Red as an encapsulated fluorescent marker) in subsequent uptake experiments performed under the same conditions (Figure 1). Therefore, it was concluded that DMPC/DMPC nanocarrier formulations were superior to neutral and pegylated formulations for delivering BDQ to the intracellular environment of macrophage-like cells and, despite a higher level of cell stress markers, which might be explained by more intensive interactions of the anionic DMPC/DMPC nanocarrier with the cells, they also appeared to reduce the number of cells with pronounced DIPL in the cell population, as compared to neutral and pegylated nanocarriers.

### Example 3: Uptake of Bedaquiline

BDQ and Texas Red encapsulated in lipid-based nanocarrier (TargoSphere^{™}) systems as described in Example 1 were determined in phosphate-buffered saline (0.1 M, pH 7.4) supplemented with 10.8 mg/mL albumin at 37°C (Patel, 2020, J Control Release. 2020 Dec 10;328:339-349). The receiver fluid was supplemented with albumin to ensure sink conditions and to mimic the protein concentration in lung lining fluid [Bicer, 2011, Drug Deliv. to Lungs 22, Edinburgh (2011)]. The nanocarrier (TargoSphere^{™}) formulations (10 mg/mL, 1 mL) were placed in dialysis tubing (Fischer, MWCO 10 kDa) and then the dialysis membranes were immersed in beakers containing 10 mL receiver fluid. The beakers were incubated at 37 °C under gentle stirring (100 strokes/min) in a water bath (Grant Instruments, Cambridge, UK). Samples of the receiver fluid were withdrawn from the beaker and replaced with fresh medium at regular intervals to maintain sink conditions. BDQ was extracted from the receiver fluid samples by mixing with dichloromethane (DCM; 10 mL), sonicating for 20 minutes followed by a 60-minute resting period for phase separation. The denser DCM layer was carefully drip-separated from the aqueous buffer phase and allowed to evaporate at room temperature overnight. The extraction process was repeated three times in total. The dried sample containing BDQ was dissolved in 1 mL DMSO and analyzed by UV spectroscopy (n=3 replicates).

As can be seen from Fig. 2, by using DMPG/DMPC nanocarriers, the uptake into the cell could be increased significantly.

### Example 4: In-vivo assessment of respirable bedaquiline-loaded nanocarriers designed for inhalation therapy of multidrug-resistant tuberculosis

The intrapulmonary administration of BDQ-loaded lipid-based nanocarriers (TargoSpheres^{™}) achieved a sufficiently increased lung concentration of the antibiotic compared to intravenous and peroral administration.

Method: BDQ-loaded nanocarriers (TargoSpheres^{™}) were administered intravenously (IV) and intranasally (IN, Southam et al., Am. J. Physiol. Lung Cell Mol. Physiol., 282 2002, L833-L839) an accepted form of intrapulmonary dosing) at a dose of 2.5 mg/kg. A suspension of unformulated BDQ crystals dispersed in a glucose solution containing carmellose was administered orally (p.o.). After 0.5, 3, 24, 48, 72 and 96 hours, the animals were sacrificed and samples were taken from the blood plasma, lung tissues, lung epithelial fluid and cellular fraction of the lung epithelial fluid. The concentrations of BDQ in the four tissue compartments were determined using LC-MS/MS.

Two comparative studies served to validate the methodology. The first publication by Rouan et al. (Antimicrob Agents Chemother. 2012 Mar;56(3):1444-51) shows the plasma concentrations of BDQ (referred to as TMC207) over a period of 168 hours after oral administration (30 mg/kg) in male Swiss mice. Irwin et al. (ACS Infect Dis. 2016 2: 251-267) showed BDQ plasma and lung concentrations over 168 hours after oral administration of 25 mg/kg in male Balb/c mice. Both studies solubilized the drug in an aqueous solution containing 20% 2-hydroxypropyl-beta-cyclodextrin prior to administration, so the rate of dissolution of the very lipophilic drug should not have any impact on the pharmacokinetics. In the current study, the active substance was administered as a suspension (25 mg/kg), since the consideration of the dissolution rate of the unprocessed BDQ particles ("neat BDQ") in the stomach better reflects the conditions of a standard-of-care dosage form (tablet).

After both i.v. and p.o. administration, BDQ is distributed in various peripheral compartments, including the lung tissue. The literature shows that BDQ accumulates in the lung with a AUCLung:AUCplasma ratio of 20 (Rouan et al., 2012). In our current study, p.o. administration resulted in a lung accumulation ratio of 16.4 and i.v. administration lung accumulation was 19.1. In contrast, i.n. administration achieved an AUCLunge:AUCplasma ratio of 91 and thus a 4.5-fold increase in lung exposure after intrapulmonary administration.

It is postulated that higher BDQ concentrations in the lungs lead locally to a steeper concentration gradient. This gradient drives drug diffusion through the lungs and is believed to increase levels of active drug in hard-to-reach compartments such as the fibrotic granuloma capsule. This should lead to an increase in therapy efficiency with a reduction in the development of resistance.

One further advantage of inhaling nebulized BDQ-nanocarriers would be bypassing the first passage through the liver (the so-called "first-pass effect") and thus delayed metabolization of the BDQ to N-desmethyl-BDQ. The encapsulation of the BDQ could further prevent or delay distribution processes such as protein and albumin binding as well as diffusion processes in other tissues, which could lead to altered biodistribution in the body and pharmacokinetics. The BDQ metabolite N-desmethyl-BDQ is primarily formed in the liver by cytochrome P450 34A biotransformation and has a 3- to 5-fold attenuated activity against Mycobacterium tuberculosis strains compared to BDQ. The metabolite is also responsible for a major proportion of systemic side effects - such as phospholipidosis and "QT prolongation".

## Claims

1. An active agent complex comprising one or more anti-bacterial agents encapsulated in a 1,2-Dimyristoyl-sn-glycero-3-phosphoglycerol (DMPG)- and/or 1,2-Dimyristoyl-sn-glycero-3-phosphocholine (DMPC)-based nanocarrier having at least one cell-specific targeting ligand on its surface, wherein the targeting ligand specifically binds to a receptor on the surface of a CRD-positive cell or CLR-positive cell of the lower airways for use in a method of treating a bacterial infection of the lower airways by inhalation.

2. The active agent complex for the use of claim 1, wherein the anti-bacterial agent is bedaquiline (BDQ), kanamycin A, amikacin, tobramycin, dibekacin, gentamicin, sisomicin, netilmicin, neomycin B, neomycin C, paromomycin, streptomycin; moxifloxacin, levofloxacin, sparfloxacin, nalidixic acid, ciprofloxacin, cinoxacin, oxolinic acid, piromidic acid, pipemidic acid, rosoxacin, enoxacin, fleroxacin, lomefloxacin, nadifloxacin, norfloxacin, ofloxacin, perfloxacin, rufloxacin, balofloxacin, grepafloxacin, pazufloxacin, temafloxacin, tosufloxacin, clinafloxacin, gatlifloxacin, sitafloxacin, prulifloxacin, delafloxacin, nemofloxacin, danofloxacin, difloxacin, enrofloxacin, ibafloxacin, marbofloxacin, orbifloxacin, sarafloxacin, trovafloxacin, linezolid, metronidazole, tinidazole and/or nimorazole.

3. The active agent complex for the use of claim 2, wherein the anti-bacterial agent is bedaquiline (BDQ).

4. The active agent complex for the use of any of claims 1-3, wherein the targeting ligand is monofucose, polyfucose, cholesten-5-yloxy-N-(4-((1-imino-2-D-thiofucosylethyl)amino)alkyl)formamide (Fuc-C4-chol) or cholesteryl-(4-(3-((2R,3S,4R,5S,6S)-3,4,5-trihydroxy-6-(hydroxymethyl)tetrahydro-2H-pyran-2-yl)thioureido)butyl)carbamate (Thiourea-Galac-C4-chol).

5. The active agent complex for the use of any of claims 1-4 , wherein the lower airways include the portion of the larynx below the vocal folds, trachea, bronchi, bronchioles and the lungs.

6. The active agent complex for the use of any of claims 1-5, wherein the bacterial infection of the lower airways comprises tuberculosis, cystic fibrosis, pneumonia and chronic obstructive pulmonary disease.

7. The active agent complex for the use of claim 6, wherein the tuberculosis is multi-drug resistant tuberculosis.

8. The active agent complex for the use of claim 6 or 7, wherein the infections is caused by one or more bacteria selected from the group consisting of *Pseudomonas aeruginosa, Pseudomonas fluorescens, Pseudomonas acidovorans, Pseudomonas alcaligenes, Pseudomonas putida, Bordetella pertussis, Bordetella parapertussis, Bordetella bronchiseptica, Haemophilus influenzae, Haemophilus parainfluenzae, Legionella pneumophila, Burkholderia cepacia, Streptococcus pneumoniae* and *Mycobacterium tuberculosis.*

9. A pharmaceutical formulation comprising
(a) an active agent complex comprising one or more anti-bacterial agents encapsulated in a 1,2-Dimyristoyl-sn-glycero-3-phosphoglycerol (DMPG)-based and/or 1,2-Dimyristoyl-sn-glycero-3-phosphocholine (DMPC)-based nanocarrier having at least one cell-specific targeting ligand on its surface, wherein the targeting ligand specifically binds to a receptor on the surface of a CRD-positive cell or CLR-positive cell of the lower airways, and
(b) a pharmaceutically acceptable carrier, diluent, excipient, buffer and/or surfactant
for use in a method of treating a bacterial infection of the lower airways by inhalation.

10. The pharmaceutical formulation for the use of claim 9, wherein the inhalation is achieved by inhaling of a nebulized suspension comprising the active agent complex through the mouth and throat.

11. The pharmaceutical formulation for the use of claim 9 or 10, wherein the anti-bacterial agent is bedaquiline (BDQ), kanamycin A, amikacin, tobramycin, dibekacin, gentamicin, sisomicin, netilmicin, neomycin B, neomycin C, paromomycin, streptomycin; moxifloxacin, levofloxacin, sparfloxacin, nalidixic acid, ciprofloxacin, cinoxacin, oxolinic acid, piromidic acid, pipemidic acid, rosoxacin, enoxacin, fleroxacin, lomefloxacin, nadifloxacin, norfloxacin, ofloxacin, perfloxacin, rufloxacin, balofloxacin, grepafloxacin, pazufloxacin, temafloxacin, tosufloxacin, clinafloxacin, gatlifloxacin, sitafloxacin, prulifloxacin, delafloxacin, nemofloxacin, danofloxacin, difloxacin, enrofloxacin, ibafloxacin, marbofloxacin, orbifloxacin, sarafloxacin, trovafloxacin, linezolid, metronidazole, tinidazole and/or nimorazole.

12. The pharmaceutical formulation for the use of claim 11, wherein the anti-bacterial agent is bedaquiline (BDQ).

13. The pharmaceutical formulation for the use of any of claims 9-12, wherein the targeting ligand is monofucose, polyfucose, cholesten-5-yloxy-N-(4-((1-imino-2-D-thiofucosylethyl)amino)alkyl)formamide (Fuc-C4-chol) or cholesteryl-(4-(3-((2R,3S,4R,5S,6S)-3,4,5-trihydroxy-6-(hydroxymethyl)tetrahydro-2H-pyran-2-yl)thioureido)butyl)carbamate (Thiourea-Galac-C4-chol).

14. The pharmaceutical formulation for the use of any of claims 9-13 , wherein the lower airways include the portion of the larynx below the vocal folds, trachea, bronchi, bronchioles and the lungs.
